# EUROPEAN PATENT SPECIFICATION

(11) **EP 3 683 297 B1**
(45) Date of publication and mention of the grant of the patent: **10.08.2022**
(21) Application number: 19848860.3
(22) Date of filing: 03.01.2019
(51) Int. Cl.: C12M 1/00, G01N 35/10, C12M 3/06

(54) **BIOLOGICAL REACTION DEVICE PROVIDED WITH MICROFLUIDIC OR NANOFLUIDIC STRUCTURE**
MIT MIKROFLUIDISCHER ODER NANOFLUIDISCHER STRUKTUR AUSGESTATTETE BIOREAKTORVORRICHTUNG
DISPOSITIF DE RÉACTION BIOLOGIQUE POURVU D'UNE STRUCTURE MICROFLUIDIQUE OU NANOFLUIDIQUE

(30) Priority: 29.11.2018 CN 201811443084
(43) Date of publication of application: 22.07.2020
(73) Proprietor: IGENESIS (SHANGHAI) Co., LTD., Shanghai 201318 (CN)
(72) Inventor: YU, Jiachang, Shanghai 201318 (CN); ZHANG, Jia, Shanghai 201318 (CN); XU, Quan, Shanghai 201318 (CN)
(74) Representative: Qip Patentanwälte Dr. Kuehn & Partner mbB
(86) International application number: PCT/CN2019/070191
(87) International publication number: WO 2020/107641

(56) References cited:
- WO-A1-2006/014140
- WO-A1-2018/022971
- WO-A2-2007/047336
- CN-A- 105 087 353
- CN-A- 108 642 140
- CN-A- 108 642 569
- CN-A- 108 823 089
- US-A1- 2013 065 280

## Description

### FIELD OF THE INVENTION

The present application relates to the technical fields of biochemistry and molecular biology, and more particularly relates to a bioreactor device equipped with a microfluidic or nanofluidic structure.

### BACKGROUND OF THE INVENTION

With the change of medical methods and continuous development of individualized medication, there is an urgent need in the field of medical examination to develop a rapid and accurate detection means. In this regard, molecular detection exhibits unique advantages. The basis of molecular detection is to detect nucleic acids (DNA and RNA) in a molecular level, by analyzing the genes and the expression products thereof from tissue cells, a hair, an anticoagulant, a dry blood trace or a formaldehyde-fixed or paraffin-embedded tissue from a person to be detected. As a result, the molecular detection is able to accurately detect a disease once the disease occurs or even before any symptoms, changes of signs or biochemistry occurs.

At present, molecular detection technologies mainly include nucleic acid hybridization, polymerase chain reaction (PCR) and biochip technology and the like. Molecular detection products are mainly used in various clinical departments to detect tumor, infection, genetics, prenatal screening and the like, and also used in physical examination centers, technical service centers, third-party testing institutions and rapid micro-testing markets. Presently, detection means for blood routine, cytology, pathology and immunology are all developing towards automation, integration and standardization. However, due to the technical complexity of molecular detection, there are very few fully automated instruments platforms that handle in a "sample-to-result" model. Alternatively, many technical problems are difficult to solve. For example, the centrifugal column method or the magnetic bead method used to extract nucleic acid in biotechnology generally comprise four steps of cleaving, binding, rinsing, and eluting as well as the subsequent detection steps such as nucleic acid hybridization, polymerase chain reaction (PCR), a biochip and the like. As a result, it is very difficult to realize a fully automated instrument in a "sample-to-result" model. In the prior art, the active ingredients are usually transferred manually when transferring of active ingredients between the steps. In other words, the raw materials or intermediate products are transferred by a person between the sequential steps or the reaction vessels for carrying out the sequential steps. Obviously, it is complicated, time-consuming and laborious for transferring the materials manually as adopted in the prior art. More importantly, the whole operations even are easy to cause contamination and influence the purities of the extracts. Furthermore, the materials are difficult to be fully mixed and efficiently transferred, which influences the experimental results. Therefore, due to the technical complexity of molecular detection, there are very few fully automated instruments platforms that handle in a "sample-to-result" model. Alternatively, many technical problems are difficult to solve. In order to realize full and efficient transferring of the materials between different steps, it is necessary to comprehensively consider the structures and functions of the consumables and experimental devices. Thus, it is possible to avoid the problems existing in the prior art of high-cost, low sensitivity, complicated structures, over-complexity to operate the related detection devices and the like.

Therefore, there is an urgent need in the field to develop a bioreactor device which has a simple structure and is convenient to operate.

US 2013/065280 A1 discloses a microfluidic apparatus and a control method thereof.

### SUMMARY OF THE INVENTION

In view of the defects existing in the prior art, a technical problem solved by the present invention is to provide a bioreactor device equipped with a microfluidic or nanofluidic structure, which connects a biological sample-extraction chamber, a metering chamber and a reaction chamber. In addition, the biological sample (e. g., protein, nucleic acid) in the biological sample-extraction chamber is accurately quantified by the microfluidic or nanofluidic structure, and sufficiently and efficiently transferred to the reaction chamber. Therefore, the experimental processes are simplified, and the experimental efficiency is improved. Furthermore, when the biological sample is transferred, the risk of contamination is reduced.

Another technical problem to be solved by the present invention is to provide a bioreactor device equipped with a microfluidic or nanofluidic structure, which has a simple structure and is convenient to operate. Furthermore, the bioreactor device can effectively simplify the equipment used in conjunction therewith.

In order to achieve the above objects, the present application provides the following technical solutions.

In a first aspect, the present application provides a bioreactor device as defined in claim 1.

In an embodiment of the first aspect, the bioreactor device further comprises a metering device for quantifying the biological sample and the reaction liquid to be biologically reacted with the biological sample, and wherein the biological sample-extraction chamber, the metering device, and the reaction chamber are in fluid communication with each other.

In an embodiment of the first aspect, the metering device comprises one or more of the followings: a metering chamber, a microfluidic or nanofluidic chip, or a microfluidic or nanofluidic capillary.

In an embodiment of the first aspect, the bioreactor device further comprises a mixing driver fixed above the microfluidic or nanofluidic structure, and the mixing driver comprises a mixing nozzle extending through the microfluidic or nanofluidic structure and inserting into the liquid level in the biological sample-extraction chamber.

In an embodiment of the first aspect, the bioreactor device further comprises at least one reaction liquid storing tank for storing the reaction liquid, and the reaction liquid storing tank is disposed above the microfluidic or nanofluidic structure and in fluid communication with the reaction chamber through the reaction liquid injection channel.

According to the first aspect, the bioreactor device further includes a decompression device, a decompression channel, and a third valve, wherein the decompression device and the decompression channel are disposed above the microfluidic or nanofluidic the structure, the decompression device is in fluid communication with the reaction chamber through the decompression channel, and the third valve is used for blocking or opening the decompression channel.

According to the first aspect, the first valve, the second valve, and the third valve are integrated into one press-type valve. When the valve button on the press-type valve is pressed, the channel of the press-type valve is in communication with the biological sample channel. As a result, the biological sample channel is opened, while the reaction liquid injection channel and the decompression channel are closed. When the valve button on the press-type valve is pulled up, the channel of the valve is deviated from the biological sample channel. As a result, the biological sample channel is closed and the reaction liquid injection channel and the decompression channel are opened, resulting in the opening of the decompression channel.

In an embodiment of the first aspect, the biological sample-extraction chamber contains magnetic beads which adsorb macromolecular extracts including nucleic acid or protein.

In an embodiment of the first aspect, the biological sample-extraction chamber further comprises an electromagnet which adsorb the magnetic beads in the biological sample-extraction chamber, wherein the electromagnet is disposed outside the biological sample-extraction chamber on the side away from the liquid delivery tip.

In an embodiment of the first aspect, when the materials in the biological sample-extraction chamber are mixed, the electromagnet is in a non-energized state, and the magnetic beads are dispersed in the biological sample-extraction chamber; when the metering chamber, the electromagnet is energized and the magnetic beads are attracted to the side away from the liquid delivery tip.

According to the first aspect, the decompression device is a decompression pump which includes a piston push-pull structure.

In an embodiment of the first aspect, the reaction liquid storing tank comprises a piston push-pull structure, wherein the piston push-pull structure is provided with a puncture structure, and a cavity for accommodating the reaction liquid is formed in the reaction liquid storing tank with an encapsulation film in the cavity bottom.

In an embodiment of the first aspect, the biological sample-extraction chamber and the reaction chamber are both removable structures.

In an embodiment of the first aspect, the biological sample-extraction chamber and the reaction chamber are fixed to the main structure of the bioreactor device equipped with the microfluidic or nanofluidic structure by ways of buckle or screw thread.

In an embodiment of the first aspect, the microfluidic or nanofluidic structure comprises a positioning via that enables fluid joining and connection.

Compared with the prior art, the beneficial effects of the present application are that the bioreactor device of the present application is equipped with the microfluidic or nanofluidic structure, wherein the microfluidic or nanofluidic structure is provided to connect the biological sample-extraction chamber, the metering chamber and the reaction chamber. In addition, the biological sample (e.g., protein, nucleic acid) in the biological sample-extraction chamber is accurately quantified by the microfluidic or nanofluidic structure, and sufficiently and efficiently transferred to the reaction chamber. Therefore, the experimental processes are simplified, and the experimental efficiency is improved. Furthermore, when the biological sample is transferred, the risk of contamination is reduced. In particular, the mixing driver is disposed on the bioreactor device of the present application which is equipped with the microfluidic or nanofluidic structure. By pushing and pulling the mixing driver, the materials in the biological sample-extraction chamber are mixed and stirred, facilitating fully eluting of the extract adsorbed around the magnetic beads. In addition, a metering pump is disposed on the bioreactor device of the present application. When the metering pump is pumped backward, the negative pressure generated in the metering pump forces the biological sample in the biological sample-extraction chamber into the metering pump through the liquid delivery tip, the biological sample channel, and the metering chamber. The reaction liquid injection channel is in fluid communication with the reaction chamber via the metering chamber. The reaction liquid in the reaction liquid storing tank is injected into the reaction chamber via the reaction liquid injection channel and the metering chamber. As a result, the biological sample (e.g., protein, nucleic acid) is sufficiently and efficiently transferred from the biological sample-extraction chamber to the reaction chamber. Therefore, the experimental processes are simplified, and the experimental efficiency is improved. Furthermore, when the biological sample is transferred, the risk of contamination is reduced.

In addition, the bioreactor device of the present invention, which is equipped with the microfluidic or nanofluidic structure, has a simple overall structure and is easy to operate. The devices disposed in the bioreactor device of the present invention, including the microfluidic or nanofluidic structure, the press-type valve, the removable chamber for biological sample extraction and the reaction chamber, the piston-type reaction liquid storing tank, the mixing driver, the metering pump and the like, are all able to collaborate very well with external automation devices, especially the pressing device or push-pull device with a cylinder or driving mechanism. Furthermore, the related external devices for implementing such simple actions are greatly simplified, thereby effectively simplifying the devices used with the bioreactor device of the present invention.

### BRIEF DESCRIPTION OF THE DRAWINGS

Figure 1 is a schematic diagram of a bioreactor device equipped with a microfluidic or nanofluidic structure according to a preferred embodiment of the present invention.
Figure 2 is another schematic diagram of a bioreactor device equipped with a microfluidic or nanofluidic structure according to a preferred embodiment of the present invention.
Figure 3 is a schematic diagram of a microfluidic or nanofluidic structure in a bioreactor device equipped with a microfluidic or nanofluidic structure according to a preferred embodiment of the present invention. In figure 3, the hollow arrow indicates the flow direction of the biological sample, and the solid arrow indicates the flow direction of the reaction liquid.
Figure 4 is a cross-sectional view of the valve region in Figure 2.
Figure 5 is another schematic diagram of a bioreactor device equipped with a microfluidic or nanofluidic structure according to a preferred embodiment of the present invention.
Figure 6 is a further schematic diagram of a bioreactor device equipped with a microfluidic or nanofluidic structure according to a preferred embodiment of the present invention.

### DETAILED DESCRIPTION OF THE PREFERRED EMBODIMENTS

The technical solutions of the present application will be clearly and completely described in the following with reference to the accompanying drawings and embodiments of the present application.

Embodiments of the present invention provide a bioreactor device equipped with a microfluidic or nanofluidic structure, which comprises a biological sample-extraction chamber, a metering chamber, a reaction chamber, and a microfluidic or nanofluidic structure for transferring a macromolecular extract in the biological sample-extraction chamber to the reaction chamber. A mixing driver, disposed above the microfluidic or nanofluidic structure, is fixed to the microfluidic or nanofluidic structure. In addition, the mixing driver has a mixing nozzle extending through the microfluidic or nanofluidic structure and inserting into the liquid level in the biological sample-extraction chamber. By pulling and pushing the mixing driver, the materials in the biological sample-extraction chamber are mixed. Furthermore, a metering pump is disposed above the microfluidic or nanofluidic structure, and a metering chamber is disposed in the microfluidic or nanofluidic structure. The microfluidic or nanofluidic structure further comprises a biological sample channel to connect the biological sample-extraction chamber and the metering chamber. The other end of the biological sample channel is in fluid communication with the liquid delivery tip disposed in the biological sample-extraction chamber. The other end of the metering chamber is in fluid communication with the channel of the metering pump. When the metering pump is pumped backward, the negative pressure generated in the metering pump forces the biological sample in the biological sample-extraction chamber into the metering pump through the liquid delivery tip, the biological sample channel, and the metering chamber. In addition, a reaction liquid storing tank is disposed above the microfluidic or nanofluidic structure. The microfluidic or nanofluidic structure further comprises reaction liquid injection channels which is in fluid communication with the reaction chamber via the metering chamber. Before the reaction starts in the reaction chamber, the reaction liquid in the reaction liquid storing tank is injected into the reaction chamber via the metering chamber through the reaction liquid injection channel. Then, the reaction liquid is mixed and reacted with the biological sample obtained from the biological sample-extraction chamber and transferred to the reaction chamber. Moreover, a decompression pump is disposed above the microfluidic or nanofluidic structure, and a decompression channel is disposed above the microfluidic or nanofluidic structure, wherein the decompression pump is in fluid communication with the reaction chamber through the decompression channel. Before the reaction liquid is injected into the reaction chamber, the decompression pump is pumped backward, and the reaction chamber generates a negative pressure so that the reaction liquid is smoothly injected into the reaction chamber.

In one embodiment, a liquid valve capable of blocking or opening the biological sample channel is disposed above the microfluidic or nanofluidic structure, wherein the valve is capable of blocking or opening the decompression channel and the decompression channel. In a preferred embodiment of the present invention, the valve is a press-type valve. After a single press of the valve button on the valve, the channel of the valve is in fluid communication with the biological sample channel so that the biological sample channel is opened. Meanwhile, the reaction liquid injection channel and the decompression channel are closed. After the valve button on the valve is pulled up, the channel of the valve is deviated from the biological sample channel so that the biological sample channel is closed. At the same time, the reaction liquid injection channel is in fluid communication with the channel of the valve so that the reaction liquid injection channel is opened. Furthermore, the decompression channel is also in fluid communication with the channel of the valve so that the decompression channel is opened.

In one embodiment, the biological sample-extraction chamber contains magnetic beads that adsorb macromolecular extracts including nucleic acid or protein.

In one embodiment, the biological sample-extraction chamber further comprises an electromagnet to adsorb the magnetic beads in the biological sample-extraction chamber, wherein the electromagnet is disposed outside at the side away from the liquid delivery tip. When the materials in the biological sample-extraction chamber are mixed, the electromagnet is in a non-energized state, and the magnetic beads are dispersed in the biological sample-extraction chamber. The materials in the biological sample-extraction chamber are mixed by pushing and pulling the mixing driver, which facilitates the efficient elution of the extract adsorbed to the magnetic beads in the biological sample-extraction chamber. When the extract is efficiently eluted and the biological sample is needed to be transferred from the biological sample-extraction chamber to the metering chamber, the electromagnet is energized and the magnetic beads are attracted to the side away from the liquid delivery tip.

In one embodiment, one or two or more reaction liquid storing tanks are provided. In a preferred embodiment of the invention, in one embodiment of the invention, there are two the reaction liquid storing tanks.

In one embodiment, the metering chamber may be a buckle-type replaceable structure.

In one embodiment, the decompression pump includes a piston push-pull structure. Before the reaction liquid is needed to be injected into the reaction chamber, the piston is pumped backward. As a result, a negative pressure is generated in the reaction chamber so that the reaction liquid is smoothly injected into the reaction chamber.

In one embodiment, the reaction liquid storing tank comprises a piston push-pull structure, wherein the piston push-pull structure is provided with a puncture structure. A cavity for accommodating the reaction liquid is formed in the reaction liquid storing tank with an encapsulation film in the cavity bottom. When the reaction liquid is needed to be injected into the reaction chamber, the piston push-pull structure is pushed. Then, the puncture structure pierces the encapsulation film at the bottom of the cavity, and the reaction liquid is injected into the reaction chamber through the reaction liquid injection channel.

In one embodiment, the biological sample-extraction chamber and the reaction chamber are both removable structures.

In one embodiment, the biological sample-extraction chamber and the reaction chamber are fixed to the main structure of the bioreactor device equipped with the microfluidic or nanofluidic structure by ways of buckle or screw thread.

The technical solutions in the embodiments of the present invention are clearly and completely described in the following with reference to the accompanying drawings in the embodiments of the present invention. It is obvious that the described embodiments are only part of the embodiments of the present invention, but do not form all embodiments of the present invention. All other embodiments, obtained by those skilled in the art based on the embodiments of the present invention without inventive efforts, are in the scope of the present invention.

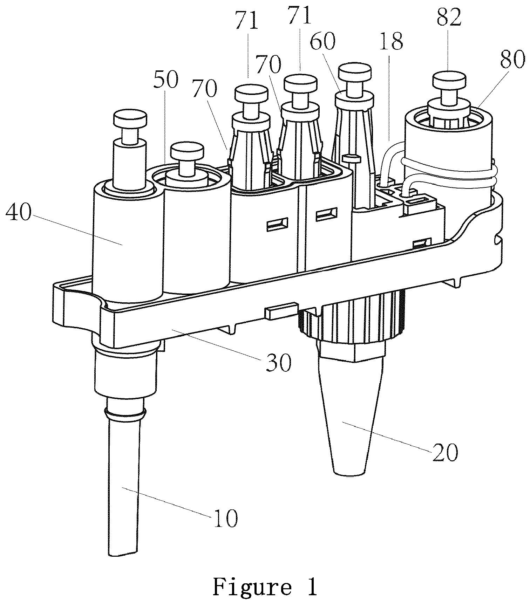

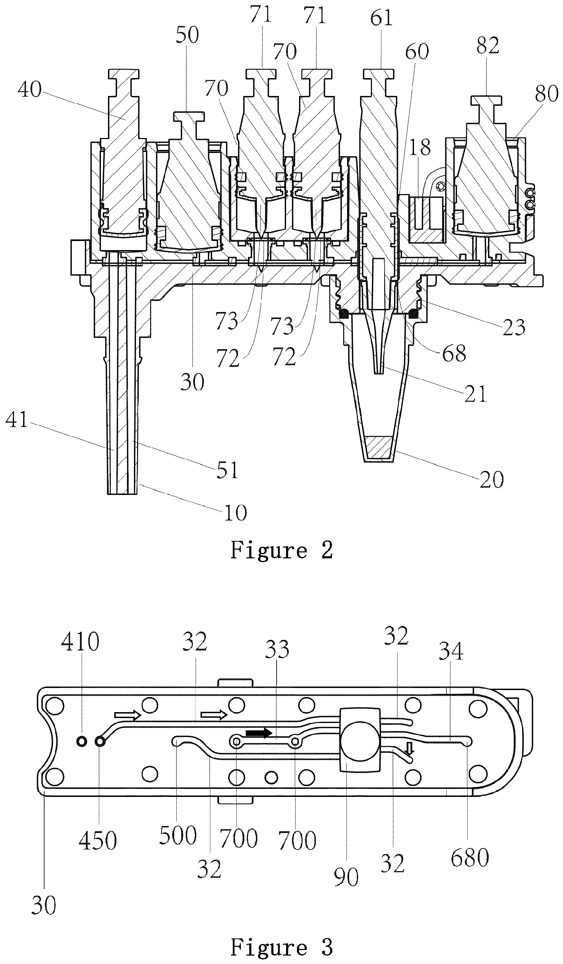

Figures 1 and 2 are schematic diagrams showing the structure of a bioreactor device equipped with a microfluidic or nanofluidic structure according to a preferred embodiment of the present invention. The bioreactor device equipped with the microfluidic or nanofluidic structure is applied to devices used for the preparation and detection of nucleic acid or protein. The bioreactor device aims to, but not limited to, provide an efficient and rapid solution for material transfer between adjacent steps in obtaining and subsequent detecting the nucleic acid or protein samples. In practical applications, the extraction cartridge can also be used to extract other biological extracts.

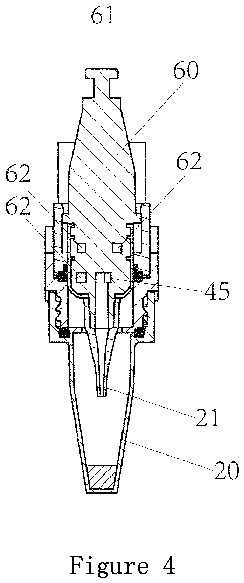

As shown in Figures 1-4, a bioreactor device 30 equipped with a microfluidic or nanofluidic structure provided by a preferred embodiment of the present invention includes a biological sample-extraction chamber 10, a metering chamber 18, a reaction chamber 20, and a microfluidic or nanofluidic structure 30 for transferring macromolecular extracts from the biological sample-extraction chamber 10 to the reaction chamber 20. A mixing driver 40 is disposed above and secured to the microfluidic or nanofluidic structure 30. The mixing driver 40 is provided with a mixing nozzle 41 extending through the microfluidic or nanofluidic structure 30 and inserting into the liquid level in the biological sample-extraction chamber 10. By pushing and pulling the mixing driver 40, materials in the biological sample-extraction chamber 10 are mixed.

Furthermore, a metering pump 50 is disposed above the microfluidic or nanofluidic structure 30, and a metering chamber 18 is disposed in the microfluidic or nanofluidic structure. In addition, the microfluidic or nanofluidic structure 30 comprises a biological sample channel 32 connecting the biological sample-extraction chamber 10 and the metering chamber 18. One end of the biological sample channel 32 is in fluid communication with a liquid delivery tip 51 disposed in the biological sample-extraction chamber 10. The other end of the metering chamber 18 is in fluid communication with the biological sample channel 32 of the metering pump 50. When the metering pump is pumped backward (i.e., a piston of the metering pump is pulled upward), the negative pressure generated by the metering pump 50 forces the biological samples in the biological sample-extraction chamber 10 to enter the metering chamber 18 through the liquid delivery tip 51 and the biological sample channel 32.

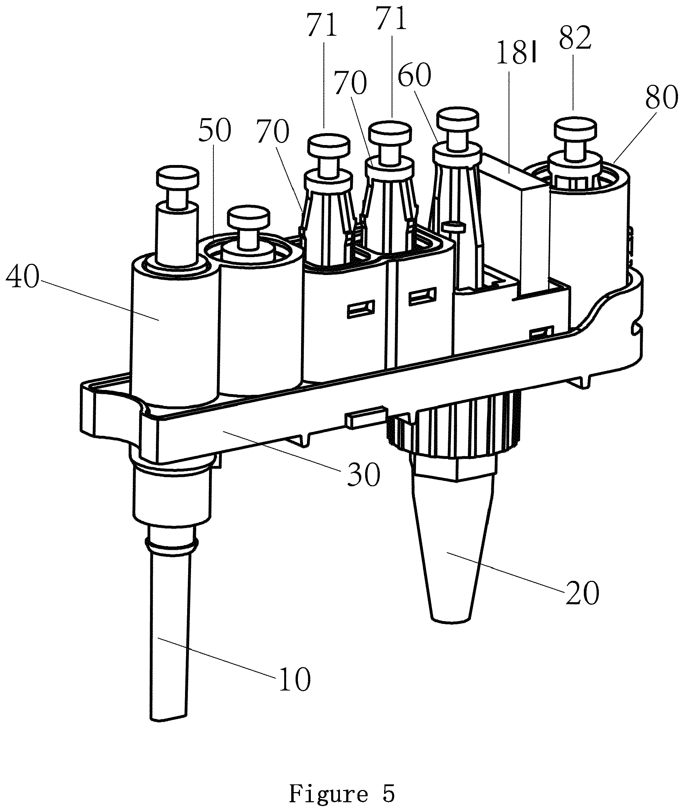

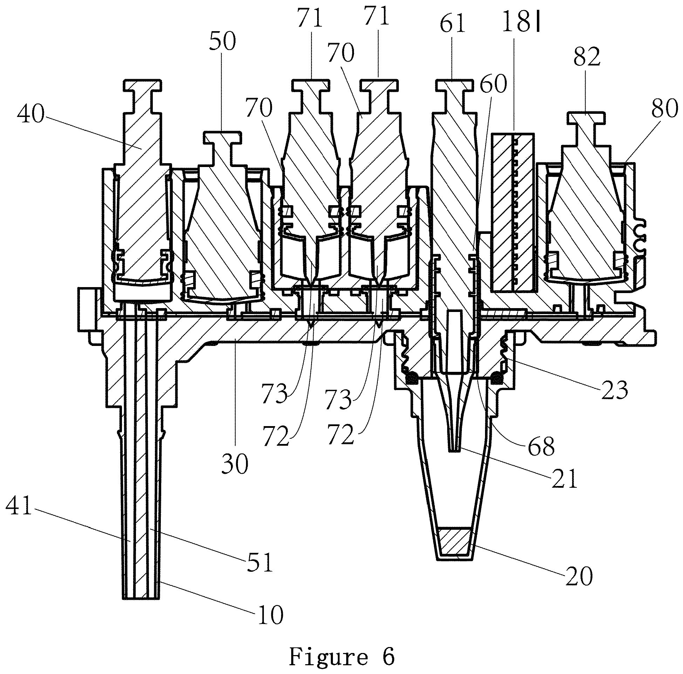

In another particular embodiment, referring to Figures 5 and 6, the metering device may be configured as a microfluidic or nanofluidic chip 181 or a microfluidic or nanofluidic capillary.

In addition, a reaction liquid storing tank 70 is disposed above the microfluidic or nanofluidic structure 30. Furthermore, a reaction liquid injection channel 33 is disposed in the microfluidic or nanofluidic structure 30. The reaction liquid injection channel 33 is in fluid communication with the reaction chamber 20 via the metering chamber 18. Before the reaction starts in the reaction chamber 20, the reaction liquid in the reaction liquid storing tank 70 is injected into the reaction chamber 20 through the reaction liquid injection channel 33 and the metering chamber 18, so as to be mixed and reacted with the biological samples obtained from the biological sample-extraction chamber 10 and transferred to the reaction chamber 20.

In addition, a decompression pump 80 with a decompression piston 82 therein are disposed above the microfluidic or nanofluidic structure 30. A decompression channel 34 is also disposed above the microfluidic or nanofluidic structure 30. The decompression pump 80 is in fluid communication with the reaction chamber 20 through the decompression channel 34. Before the reaction liquid is injected into the reaction chamber 20, the decompression piston 82 in the decompression pump 80 is pumped backward. Consequently, the reaction chamber 20 generates a negative pressure so that the reaction liquid is smoothly injected into the reaction chamber 20.

In a preferred embodiment of the invention, the microfluidic or nanofluidic structure 30 is further provided with a liquid valve 60 capable of blocking or opening the decompression channel 34. In addition, the valve 60 is capable of blocking or opening the reaction liquid injection channel 33. Specifically, the valve 60 is a press-type valve 60. After pressing the valve button 61 on the valve 60, the channel of the valve 62 is in fluid communication with the biological sample channel 32, and the biological sample channel 32 is opened. Meanwhile, the reaction liquid injection channel 33 and the pressure-reducing channel 34 are closed. After pulling up the valve button 61 on the valve 60, the channel of the valve 62 is deviated from the biological sample channel 32, and the biological sample channel 32 is closed. Meanwhile, the reaction liquid injection channel 33 and the pressure reduction channel 34 are opened.

In a specific embodiment, the biological sample channel has a width of 0. 55 to 0. 75 mm, a depth of 0. 50 to 0. 65 mm, and a length of 8 to 12 mm. In a preferred embodiment, the biological sample channel has a width of 0. 65 mm, a depth of 0.6 mm, and a length of 12 mm.

In a specific embodiment, the reaction liquid channel has a width of 0.55 to 0.75 mm, a depth of 0.50 to 0.65 mm, and a length of 8 to 12 mm. In a preferred embodiment, the reaction liquid channel has a width of 0. 65 mm, a depth of 0.6 mm, and a length of 12 mm.

In a preferred embodiment of the invention, the biological sample-extraction chamber 10 contains magnetic beads which can adsorb macromolecular extracts including nucleic acid or protein.

In addition, the biological sample-extraction chamber 10 further comprises an electromagnet for adsorbing the magnetic beads in the biological sample-extraction chamber 10, wherein the electromagnet is disposed outside the biological sample-extraction chamber 10 on the side away from the liquid delivery tip 51. The materials in the biological sample-extraction chamber 10 are mixed by pushing and pulling the mixing driver 40, to facilitate the efficient elution of the extracts adsorbed by the magnetic beads in the biological sample-extraction chamber 10, the electromagnet is in a non-energized state, and the magnetic beads are dispersed in the biological sample-extraction chamber 10. When the biological sample is required to be transferred from the biological sample-extraction chamber 10 to the metering chamber 18 for the efficient elution of the extracts, the electromagnet is energized and the magnetic beads are attracted to the side away from the liquid delivery tip 51. In addition, the biological sample (e.g., protein, nucleic acid) in the biological sample-extraction chamber 10 is accurately quantified and efficiently transferred to the reaction chamber 20 in total by the microfluidic or nanofluidic structure 30. Thereby, the magnetic beads and other impurities are prevented to be transferred together with the biological sample (e.g., protein, nucleic acid) while it is fully and efficiently transferred from the biological sample-extraction chamber 10 to the reaction chamber 20.

As a preferred embodiment of the present invention, in the embodiment of the present invention, two reaction liquid storing tanks 70 are provided. Certainly, in other embodiments of the present invention, one or other number of reaction liquid storing tanks 70 are provided, as long as the corresponding biochemical reaction in the reaction chamber of the present invention could be carried out.

In addition, in the embodiment of the present invention, the reaction liquid storing tank 70 includes a piston push-pull structure 71, wherein the piston push-pull structure 71 is provided with a puncture structure 73. A cavity for accommodating the reaction liquid is formed in the reaction liquid storing tank 70, and the bottom of the cavity is an encapsulation film 72. When it is required to inject the reaction liquid into the reaction chamber 20, the piston push-pull structure 71 is pushed. Consequently, the puncture structure 73 pierces the encapsulation film 72 at the bottom of the cavity, and the reaction liquid is injected into the reaction chamber 20 through the reaction liquid injection channel 33 and the metering chamber 18.

The biological sample-extraction chamber 10 and the reaction chamber 20 in the embodiment of the present invention are both removable structures, so as to facilitate installment and replacement. In a specific embodiment, the biological sample-extraction chamber 10 and the reaction chamber 20 are secured to the main structure of the bioreactor device equipped with a microfluidic or nanofluidic structure by means of screw thread 23. Certainly, in other embodiments of the present invention, the biological sample-extraction chamber 10 and the reaction chamber 20 may be fixed to the main structure of the bioreactor device by buckles or other commonly used removable fixing means.

In embodiments of the present invention, in order to scientifically arrange the microfluidic or nanofluidic structure 30 and other corresponding structures to achieve good connection and engagement of fluid flows, positioning regions and connecting micropores are disposed on the microfluidic or nanofluidic structure 30 for the related structures. so as The mixing driver 40 is fixed to the microfluidic or nanofluidic structure 30 and has a mixing nozzle 41 extending through the microfluidic or nanofluidic structure 30 and inserting into the liquid level in the biological sample-extraction chamber 10. A positioning hole 410 corresponds to the mixing driver 40. The liquid delivery tip 51, installed below the liquid level in the biological sample-extraction chamber 10 of the microfluidic or nanofluidic structure 30, is in fluid communication with the biological sample channel 32 and a valve chamber 90 through the positioning hole 450. After the pressure generated by the metering pump 50 applied to the biological samples in the biological sample-extraction chamber 10, the biological samples flow outward to the biological sample channel 32 through the liquid delivery tip 51 and then enter into the metering chamber 18. The biological sample channel 32 is in fluid communication with a liquid conduit 21 through the valve liquid-guiding port 45. The metering pump 50 is in fluid communication with the biological sample channel 32 by a positioning hole 500.

Before a reaction starts in the reaction chamber 20, a decompression chamber 80 installed in the microfluidic or nanofluidic structure 30 is in fluid communication with the reaction chamber 20 through a micropore 680, the decompression channels 34, and a decompression hole 68 on the valve 60. After the reaction chamber 20 is depressurized by the decompression piston 82 in the decompression chamber 80, a reaction liquid in the reaction liquid storing tank 70 is injected into the reaction chamber 20 through the reaction liquid injection channel 33, the metering chamber 18, and the liquid conduit 21. A micropore 700 is used to connect the reaction liquid injection channel 33, the metering chamber 18, and the liquid conduit 21.

The bioreactor device disclosed in the above embodiments is equipped with the microfluidic or nanofluidic structure, wherein the microfluidic or nanofluidic structure is provided to connect the biological sample-extraction chamber, the metering chamber and the reaction chamber. In addition, the biological sample (e.g., protein, nucleic acid) in the biological sample-extraction chamber is accurately quantified by the microfluidic or nanofluidic structure, and sufficiently and efficiently transferred to the reaction chamber. Therefore, the experimental processes are simplified, and the experimental efficiency is improved. Furthermore, when the biological sample is transferred, the risk of contamination is reduced. In particular, the mixing driver is disposed on the bioreactor device of the present application which is equipped with a microfluidic or nanofluidic structure. By pushing and pulling the mixing driver, the materials in the biological sample-extraction chamber are mixed and stirred, facilitating fully eluting of the extract adsorbed around the magnetic beads. In addition, the metering pump is disposed on the bioreactor device of the present application. When the metering pump is pumped backward, the negative pressure generated in the metering pump forces the biological sample in the biological sample-extraction chamber into the metering pump through the liquid delivery tip, the biological sample channel, and the metering chamber. The reaction liquid injection channel is in fluid communication with the reaction chamber via the metering chamber. The reaction liquid in the reaction liquid storing tank is injected into the reaction chamber via the reaction liquid injection channel and the metering chamber. As a result, the biological sample (e.g., protein, nucleic acid) is sufficiently and efficiently transferred from the biological sample-extraction chamber to the reaction chamber. Therefore, the experimental processes are simplified, and the experimental efficiency is improved. Furthermore, when the biological sample is transferred, the risk of contamination is reduced.

In addition, the bioreactor device of the present invention, which is equipped with the microfluidic or nanofluidic structure, has a simple structure and is easy to operate. The devices disposed in the bioreactor device of the present invention, including the microfluidic or nanofluidic structure, the press-type valve, the removable chamber for biological sample extraction and the reaction chamber, the piston-type reaction liquid storing tank, the mixing driver, the quantification pump and the like, are all able to collaborate very well with external automation devices, especially the pressing device or push-pull device with a cylinder or driving mechanism. Furthermore, the related external devices for implementing such simple actions are greatly simplified, thereby effectively simplifying the devices used with the bioreactor device of the present invention.

## Claims

1. A bioreactor device equipped with a microfluidic or nanofluidic structure, comprising:
a biological sample-extraction chamber for providing a biological sample to participate in a biological reaction, and comprising a liquid delivery tip disposed in the biological sample-extraction chamber;
a reaction chamber for causing the biological sample biologically react with a reaction liquid to be biologically reacted with the biological sample;
a microfluidic or nanofluidic structure for transferring the biological sample from the biological sample-extraction chamber to the reaction chamber;
wherein the microfluidic or nanofluidic structure comprises a biological sample channel and a reaction liquid injection channel;
wherein the biological sample-extraction chamber and the reaction chamber are in fluid communication with each other via the biological sample channel, and the reaction liquid is injected into the reaction chamber through the reaction liquid injection channel;
wherein the microfluidic or nanofluidic structure further comprises a first valve for blocking or opening the biological sample channel and a second valve for blocking or opening the reaction liquid injection channel;
wherein the bioreactor device further includes a decompression device, a decompression channel, and a third valve; wherein the decompression device and the decompression channel are disposed above the microfluidic or nanofluidic structure; wherein the decompression device is in fluid communication with the reaction chamber through the decompression channel; and wherein the third valve is used for blocking or opening the decompression channel;
wherein the first valve, the second valve, and the third valve are integrated into one press-type valve; wherein when the valve button on the press-type valve is pressed, the channel of the press-type valve is in fluid communication with the biological sample channel with the biological sample channel being opened while the reaction liquid injection channel and the decompression channel being closed; and when the valve button on the press-type valve is pulled up, the channel of the valve is deviated from the biological sample channel, the biological sample channel is closed while the reaction liquid injection channel and the pressure reduction channel are opened;
wherein the decompression device is a decompression pump which includes a piston push-pull structure.

2. The bioreactor device equipped with a microfluidic or nanofluidic structure of claim 1, wherein the bioreactor device further comprises a metering device for quantifying the biological sample and the reaction liquid to be biologically reacted with the biological sample, and wherein the biological sample-extraction chamber, the metering device, and the reaction chamber are in fluid communication with each other.

3. The bioreactor device equipped with a microfluidic or nanofluidic structure of claim 2, wherein the metering device comprises one or more of the followings: a metering chamber, a microfluidic or nanofluidic chip, or a microfluidic or nanofluidic capillary.

4. The bioreactor device equipped with a microfluidic or nanofluidic structure of claim 1, wherein the bioreactor device further comprises a mixing driver, and wherein the mixing driver is fixed above the microfluidic or nanofluidic structure, and comprises a mixing nozzle extending through the microfluidic or nanofluidic structure and inserting into the liquid level in the biological sample-extraction chamber.

5. The bioreactor device equipped with a microfluidic or nanofluidic structure of claim 1, wherein the bioreactor device further comprises at least one reaction liquid storing tank for storing the reaction liquid, and wherein the reaction liquid storing tank is disposed above the microfluidic or nanofluidic structure and in fluid communication with the reaction chamber through the reaction liquid injection channel.

6. The bioreactor device equipped with a microfluidic or nanofluidic structure of claim 1, wherein the biological sample-extraction chamber contains magnetic beads which adsorb macromolecular extracts including nucleic acid or protein.

7. The bioreactor device equipped with a microfluidic or nanofluidic structure of claim 6, wherein the biological sample-extraction chamber further comprises an electromagnet to adsorb the magnetic beads in the biological sample-extraction chamber, and wherein the electromagnet is disposed outside the biological sample-extraction chamber on the side away from the liquid delivery tip.

8. The bioreactor device equipped with a microfluidic or nanofluidic structure of claim 7, wherein when the materials in the biological sample-extraction chamber are mixed, the electromagnet is in a non-energized state, and the magnetic beads are dispersed in the biological sample-extraction chamber; and wherein when the biological sample is needed to be transferred from the biological sample-extraction chamber to the metering chamber, the electromagnet is energized and the magnetic beads are attracted to the side away from the liquid delivery tip.

9. The bioreactor device equipped with a microfluidic or nanofluidic structure of claim 5, wherein the reaction liquid storing tank comprises a piston push-pull structure, and wherein the piston push-pull structure is provided with a puncture structure, and a cavity for accommodating the reaction liquid is formed in the reaction liquid storing tank with an encapsulation film in the cavity bottom.

10. The bioreactor device equipped with a microfluidic or nanofluidic structure of claim 1, wherein the biological sample-extraction chamber and the reaction chamber are both removable structures.

11. The bioreactor device equipped with a microfluidic or nanofluidic structure of claim 10, wherein the biological sample-extraction chamber and the reaction chamber are fixed to the main structure of the bioreactor device equipped with a microfluidic or nanofluidic structure by ways of buckle or screw thread.

12. The bioreactor device equipped with a microfluidic or nanofluidic structure of claim 1, wherein the microfluidic or nanofluidic structure comprises a positioning via that enables fluid joining and connection.

## Patentansprüche

1. Mit einer mikrofluidischen oder nanofluidischen Struktur ausgestattete Bioreaktorvorrichtung, umfassend:
eine Kammer zur Extraktion einer biologischen Probe zur Bereitstellung einer biologischen Probe, um sich an einer biologischen Reaktion zu beteiligen, und umfassend eine Flüssigkeitsabgabespitze, die in der Kammer zur Extraktion der biologischen Probe angeordnet ist;
eine Reaktionskammer zum Bewirken, dass die biologische Probe mit einer Reaktionsflüssigkeit, die mit der biologischen Probe zu einer biologischen Reaktion gebracht werden soll, biologisch reagiert;
eine mikrofluidische oder nanofluidische Struktur zum Überführen der biologischen Probe von der Kammer zur Extraktion der biologischen Probe zur Reaktionskammer;
wobei die mikrofluidische oder nanofluidische Struktur einen Kanal für die biologische Probe und einen Kanal zur Injektion von Reaktionsflüssigkeit umfasst;
wobei die Kammer zur Extraktion der biologischen Probe und die Reaktionskammer über den Kanal für die biologische Probe in fluidischer Verbindung miteinander sind und die Reaktionsflüssigkeit durch den Kanal zur Injektion der Reaktionsflüssigkeit in die Reaktionskammer injiziert wird;
wobei die mikrofluidische oder nanofluidische Struktur weiterhin ein erstes Ventil zum Absperren oder Öffnen des Kanals für die biologische Probe und ein zweites Ventil zum Absperren oder Öffnen des Kanals zur Injektion der Reaktionsflüssigkeit umfasst;
wobei die Bioreaktorvorrichtung weiterhin eine Dekompressionsvorrichtung, einen Dekompressionskanal und ein drittes Ventil umfasst; wobei die Dekompressionsvorrichtung und der Dekompressionskanal oberhalb der mikrofluidischen oder nanofluidischen Struktur angeordnet sind; wobei die Dekompressionsvorrichtung über den Dekompressionskanal in fluidischer Verbindung mit der Reaktionskammer ist; und wobei das dritte Ventil zum Absperren oder Öffnen des Dekompressionskanals verwendet wird;
wobei das erste Ventil, das zweite Ventil und das dritte Ventil in ein Ventil vom Pressentyp integriert sind; wobei, wenn der Ventilknopf an dem Ventil vom Pressentyp gedrückt ist, der Kanal des Ventils vom Pressentyp in fluidischer Verbindung mit dem Kanal für die biologische Probe ist, wobei der Kanal für die biologische Probe geöffnet ist, während der Kanal zur Injektion der Reaktionsflüssigkeit und der Dekompressionskanal geschlossen sind; und, wenn der Ventilknopf an dem Ventil vom Pressentyp hochgezogen ist, der Kanal des Ventils von dem Kanal für die biologische Probe abgelenkt ist, der Kanal für die biologische Probe geschlossen ist, während der Kanal zur Injektion der Reaktionsflüssigkeit und der Kanal zur Druckminderung geöffnet sind;
wobei die Dekompressionsvorrichtung eine Dekompressionspumpe, die einen kolbenartigen Druck-Zug-Aufbau umfasst, ist.

2. Mit einer mikrofluidischen oder nanofluidischen Struktur ausgestattete Bioreaktorvorrichtung nach Anspruch 1, wobei die Bioreaktorvorrichtung weiterhin eine Dosiervorrichtung zur Quantifizierung der biologischen Probe und der Reaktionsflüssigkeit, die mit der biologischen Probe zu einer biologischen Reaktion gebracht werden soll, umfasst und wobei die Kammer zur Extraktion der biologischen Probe, die Dosiervorrichtung und die Reaktionskammer in fluidischer Verbindung miteinander sind.

3. Mit einer mikrofluidischen oder nanofluidischen Struktur ausgestattete Bioreaktorvorrichtung nach Anspruch 2, wobei die Dosiervorrichtung eines oder mehrere von Folgenden umfasst: eine Dosierkammer, einen mikrofluidischen oder nanofluidischen Chip oder eine mikrofluidische oder nanofluidische Kapillare.

4. Mit einer mikrofluidischen oder nanofluidischen Struktur ausgestattete Bioreaktorvorrichtung nach Anspruch 1, wobei die Bioreaktorvorrichtung weiterhin einen Mischantrieb umfasst und wobei der Mischantrieb oberhalb der mikrofluidischen oder nanofluidischen Struktur fixiert ist und eine Mischdüse, die sich durch die mikrofluidische oder nanofluidische Struktur hindurch erstreckt und in den Flüssigkeitsspiegel in der Kammer zur Extraktion der biologischen Probe einfährt, umfasst.

5. Mit einer mikrofluidischen oder nanofluidischen Struktur ausgestattete Bioreaktorvorrichtung nach Anspruch 1, wobei die Bioreaktorvorrichtung weiterhin mindestens einen Reaktionsflüssigkeitsvorratsbehälter zur Aufbewahrung der Reaktionsflüssigkeit umfasst, und wobei der Reaktionsflüssigkeitsvorratsbehälter oberhalb der mikrofluidischen oder nanofluidischen Struktur angeordnet ist und über den Kanal zur Injektion der Reaktionsflüssigkeit in fluidischer Verbindung mit der Reaktionskammer ist.

6. Mit einer mikrofluidischen oder nanofluidischen Struktur ausgestattete Bioreaktorvorrichtung nach Anspruch 1, wobei die Kammer zur Extraktion der biologischen Probe magnetische Kügelchen, die Nucleinsäure oder Protein umfassende makromolekulare Extrakte adsorbieren, enthält.

7. Mit einer mikrofluidischen oder nanofluidischen Struktur ausgestattete Bioreaktorvorrichtung nach Anspruch 6, wobei die Kammer zur Extraktion der biologischen Probe weiterhin einen Elektromagneten zum Adsorbieren der magnetischen Kügelchen in der Kammer zur Extraktion der biologischen Probe umfasst und wobei der Elektromagnet außerhalb der Kammer zur Extraktion der biologischen Probe an der von der Flüssigkeitsabgabespitze abgewandten Seite angeordnet ist.

8. Mit einer mikrofluidischen oder nanofluidischen Struktur ausgestattete Bioreaktorvorrichtung nach Anspruch 7, wobei, wenn die Materialien in der Kammer zur Extraktion der biologischen Probe gemischt werden, der Elektromagnet in einem stromlosen Zustand ist und die magnetischen Kügelchen in der Kammer zur Extraktion der biologischen Probe dispergiert sind; und wobei, wenn die biologische Probe von der Kammer zur Extraktion der biologischen Probe zur Dosierkammer überführt werden muss, der Elektromagnet mit Strom versorgt wird und die magnetischen Kügelchen zu der von der Flüssigkeitsabgabespitze abgewandte Seite angezogen werden.

9. Mit einer mikrofluidischen oder nanofluidischen Struktur ausgestattete Bioreaktorvorrichtung nach Anspruch 5, wobei der Reaktionsflüssigkeitsvorratsbehälter einen kolbenartigen Druck-Zug-Aufbau umfasst und wobei der kolbenartige Druck-Zug-Aufbau mit einer Durchstichstruktur ausgestattet ist und ein Hohlraum zum Aufnehmen der Reaktionsflüssigkeit in dem Reaktionsflüssigkeitsvorratsbehälter mit einer Einkapselungsfolie im untersten Teil des Hohlraums ausgebildet ist.

10. Mit einer mikrofluidischen oder nanofluidischen Struktur ausgestattete Bioreaktorvorrichtung nach Anspruch 1, wobei die Kammer zur Extraktion der biologischen Probe und die Reaktionskammer beide abnehmbare Strukturen sind.

11. Mit einer mikrofluidischen oder nanofluidischen Struktur ausgestattete Bioreaktorvorrichtung nach Anspruch 10, wobei die Kammer zur Extraktion der biologischen Probe und die Reaktionskammer an den Hauptaufbau der mit einer mikrofluidischen oder nanofluidischen Struktur ausgestatteten Bioreaktorvorrichtung mittels eines Schnapp- oder Gewindeanschlusses fixiert sind.

12. Mit einer mikrofluidischen oder nanofluidischen Struktur ausgestattete Bioreaktorvorrichtung nach Anspruch 1, wobei die mikrofluidische oder nanofluidische Struktur eine Positionierungsdurchlass umfasst, welcher eine Fluidzusammenführung und -verbindung ermöglicht.

## Revendications

1. Dispositif de bioréacteur équipé avec une structure microfluidique ou nanofluidique, comprenant :
une chambre d'extraction d'échantillon biologique pour fournir un échantillon biologique pour participer dans une réaction biologique, et comprenant une pointe de délivrance de liquide disposée dans la chambre d'extraction d'échantillon biologique ;
une chambre de réaction pour entraîner l'échantillon biologique à réagir avec un liquide réactionnel pour être mis à réagir biologiquement avec l'échantillon biologique ;
une structure microfluidique ou nanofluidique pour transférer l'échantillon biologique de la chambre d'extraction d'échantillon biologique à la chambre de réaction ;
la structure microfluidique ou nanofluidique comprenant un canal d'échantillon biologique et un canal d'injection de liquide réactionnel ;
la chambre d'extraction d'échantillon biologique et la chambre de réaction étant en communication fluide l'une avec l'autre via le canal d'échantillon biologique et le liquide réactionnel étant injecté dans la chambre de réaction par le canal d'injection de liquide réactionnel ;
la structure microfluidique ou nanofluidique comprenant en outre une première vanne pour bloquer ou ouvrir le canal d'échantillon biologique et une deuxième vanne pour bloquer ou ouvrir le canal d'injection de liquide réactionnel ;
le dispositif de bioréacteur comprenant en outre un dispositif de décompression, un canal de décompression et une troisième vanne ; le dispositif de décompression et le canal de décompression étant disposés au-dessus de la structure microfluidique ou nanofluidique ; le dispositif de décompression étant en communication fluide avec la chambre de réaction par le canal de décompression ; et la troisième vanne étant utilisée pour bloquer ou ouvrir le canal de décompression ;
la première vanne, la deuxième vanne et la troisième vanne étant intégrées dans une vanne de type presse ; quand le bouton de vanne sur la vanne de type presse est pressé, le canal de la vanne de type presse étant en communication fluide avec le canal d'échantillon biologique avec le canal d'échantillon biologique étant ouvert alors que le canal d'injection de liquide réactionnel et le canal de décompression sont fermés ; et quand le bouton de vanne sur la vanne de type presse est remonté, le canal de la vanne est dévié du canal d'échantillon biologique, le canal d'échantillon biologique est fermé alors que le canal d'injection de liquide réactionnel et le canal de réduction de pression sont ouverts ;
le dispositif de décompression étant une pompe de décompression qui comprend une structure de type piston à pression-traction.

2. Dispositif de bioréacteur équipé avec une structure microfluidique ou nanofluidique selon la revendication 1, dans lequel le dispositif de bioréacteur comprend en outre un dispositif de dosage pour quantifier l'échantillon biologique et le liquide réactionnel à être mis à réagir avec l'échantillon biologique, et la chambre d'extraction d'échantillon biologique, le dispositif de dosage et la chambre de réaction étant en communication fluide l'un avec l'autre.

3. Dispositif de bioréacteur équipé avec une structure microfluidique ou nanofluidique selon la revendication 2, dans lequel le dispositif de dosage comprend un ou plusieurs de ce qui suit : une chambre de dosage, une puce microfluidique ou nanofluidique ou un capillaire microfluidique ou nanofluidique.

4. Dispositif de bioréacteur équipé avec une structure microfluidique ou nanofluidique selon la revendication 1, dans lequel le dispositif de bioréacteur comprend en outre un moteur de mélange et le moteur de mélange étant fixé au-dessus de la structure microfluidique ou nanofluidique et comprenant une buse de mélange s'étendant à travers la structure microfluidique ou nanofluidique et s'insérant dans le niveau de liquide dans la chambre d'extraction d'échantillon biologique.

5. Dispositif de bioréacteur équipé avec une structure microfluidique ou nanofluidique selon la revendication 1, dans lequel le dispositif de bioréacteur comprend en outre au moins un réservoir de stockage de liquide réactionnel pour stocker le liquide réactionnel, et le réservoir de stockage de liquide réactionnel étant disposé au-dessus de la structure microfluidique ou nanofluidique et en communication fluide avec la chambre de réaction par le canal d'injection de liquide réactionnel.

6. Dispositif de bioréacteur équipé avec une structure microfluidique ou nanofluidique selon la revendication 1, dans lequel la chambre d'extraction d'échantillon biologique contient des billes magnétiques qui adsorbent des extraits macromoléculaires comprenant un acide nucléique ou une protéine.

7. Dispositif de bioréacteur équipé avec une structure microfluidique ou nanofluidique selon la revendication 6, dans lequel la chambre d'extraction d'échantillon biologique comprend en outre un électroaimant pour adsorber les billes magnétiques dans la chambre d'extraction d'échantillon biologique et l'électroaimant étant disposé à l'extérieur de la chambre d'extraction d'échantillon biologique sur le côté à l'écart de la pointe de délivrance de liquide.

8. Dispositif de bioréacteur équipé avec une structure microfluidique ou nanofluidique selon la revendication 7, dans lequel quand les matières dans la chambre d'extraction d'échantillon biologique sont mélangées, l'électroaimant est dans un état non énergisé, et les billes magnétiques sont dispersées dans la chambre d'extraction d'échantillon biologique ; et quand l'échantillon biologique est nécessaire pour être transféré de la chambre d'extraction d'échantillon biologique à la chambre de dosage, l'électroaimant est énergisé et les billes magnétiques sont attirées vers le côté à l'écart de la pointe de délivrance de liquide.

9. Dispositif de bioréacteur équipé avec une structure microfluidique ou nanofluidique selon la revendication 5, dans lequel le réservoir de stockage de liquide réactionnel comprend une structure de piston à poussée-traction, et la structure de piston à poussée-traction est munie avec une structure de ponction et une cavité pour loger le liquide réactionnel est formée dans le réservoir de stockage de liquide réactionnel avec un film d'encapsulation dans la partie inférieure de la cavité.

10. Dispositif de bioréacteur équipé avec une structure microfluidique ou nanofluidique selon la revendication 1, dans lequel la chambre d'extraction d'échantillon biologique et la chambre de réaction sont toutes les deux des structures enlevables.

11. Dispositif de bioréacteur équipé avec une structure microfluidique ou nanofluidique selon la revendication 10, dans lequel la chambre d'extraction d'échantillon biologique et la chambre de réaction sont fixées à la structure principale du dispositif de bioréacteur équipé avec une structure microfluidique ou nanofluidique au moyen d'une boucle ou d'un filet de vis.

12. Dispositif de bioréacteur équipé avec une structure microfluidique ou nanofluidique selon la revendication 1, dans lequel la structure microfluidique ou nanofluidique comprend une passage de positionnement qui permet la jonction et la connexion du fluide.
